# EUROPEAN PATENT SPECIFICATION

(11) **EP 3 292 867 B1**
(45) Date of publication and mention of the grant of the patent: **15.05.2019**
(21) Application number: 16188201.4
(22) Date of filing: 09.09.2016
(51) Int. Cl.: A61K 31/519, C07D 487/04, A61P 31/04, A01N 43/90, A61L 27/54

(54) **TRIAZOLO(4,5-D)PYRIMIDINE DERIVATIVES FOR USE IN THE PREVENTION AND TREATMENT OF BACTERIAL INFECTION**
TRIAZOLO(4,5-D)PYRIMIDIN-DERIVATE ZUR VORBEUGUNG UND BEHANDLUNG BAKTERIELLER INFEKTIONEN
DÉRIVÉS TRIAZOLO(4,5-D)PYRIMIDINES POUR LA PREVENTION ET LE TRAITEMENT D'INFECTIONS BACTERIENNES

(43) Date of publication of application: 14.03.2018
(73) Proprietor: Université de Liège, 4000 Liège (BE)
(72) Inventor: OURY, Cécile, 4000 LIEGE (BE); LANCELLOTTI, Patrizio, 4000 Liege (BE)
(74) Representative: Bosch, Henry

(56) References cited:
- WO-A1-00/34283
- WO-A1-2009/034386
- WO-A1-2013/092900

## Description

The present invention relates to a new use of Triazolo(4,5-d)pyrimidine derivatives for prevention and treatment of bacterial infection.

Bacteria are often incriminated in healthcare-associated infections (including medical device-related infections), causing increased patient morbidity and mortality, and posing huge financial burden on healthcare services. The situation has become critical since more and more bacteria are becoming resistant to antibiotics belonging to various classes such as Penicillins, Methicillins, Carbapenems, Cephalosporins, Quinolones, Amino-glycosides, and Glycopeptides, and an increasing number of infections are becoming difficult to cure.

The increasing resistance to antibiotics is a growing public health concern because of the limited treatment options available for these serious infections. In Europe, antimicrobial resistance causes approximately 25,000 deaths every year. The clinical burden associated with antimicrobial resistance is estimated to cost approximately €1.5 billion per year.

At present, 700,000 deaths are estimated to be attributed to antimicrobial resistance globally as reported in Review on AMR, Antimicrobial resistance: Tackling a crisis for the health and wealth of nations, 2014

The use of antibiotics is not safe especially in long-term therapy or high dose therapy. Such environmental pressure may promote selection of resistant bacteria, population, altering population structure and increasing the risk of horizontal gene transfer leading to the mobility of resistant genes into the microbiome.

Antibiotic treatment targets both the « good » and the « bad » bacteria.

The human gastro-intestinal tract (GI) microbiota is made of about trillions of microorganisms most of them bacteria. Microbiota and host's defense relationship is essential for metabolic and physiological functions contributing to health. By disrupting this benefit interaction, dietary components, physical and psychological stress, drugs but also antibiotics increase incidence of several diseases like obesity, inflammation and cardiovascular diseases (CVD). CVD remain the first cause of death in industrial society with growing incidence in other countries.

For instance recent studies showed a direct link between long term antibiotics treatment, disruption of GI microbiota and risks of atherosclerosis in mice.

The source of bacterial infection is diverse and there is a large number of bacterial infections.

Infections caused by Gram-positive bacteria represent a major public health burden, not just in terms of morbidity and mortality, but also in terms of increased expenditure on patient management and implementation of infection control measures. *Staphylococcus aureus* and *enterococci* are established pathogens in the hospital environment, and their frequent multidrug resistance complicates therapy.

*Staphylococcus aureus* is an important pathogen responsible for a broad range of clinical manifestations ranging from relatively benign skin infections to life-threatening conditions such as endocarditis and osteomyelitis. It is also a commensal bacterium (colonizing approximately 30 percent of the human population).

Two major shifts in *S*. *aureus* epidemiology have occurred since the 1990s: an epidemic of community-associated skin and soft tissue infections (largely driven by specific methicillin-resistant *S*. *aureus* [MRSA] strains), and an increase in the number of healthcare-associated infections (especially infective endocarditis and prosthetic device infections).

Coagulase-negative staphylococci (CoNS) are the most frequent constituent of the normal flora of the skin. These organisms are common contaminants in clinical specimens as well as increasingly recognized as agents of clinically significant infection, including bacteremia and endocarditis. Patients at particular risk for CoNS infection include those with prosthetic devices, pacemakers, intravascular catheters, and immunocompromised hosts.

Coagulase-negative staphylococci account for approximately one-third of bloodstream isolates in intensive care units, making these organisms the most common cause of nosocomial bloodstream infection.

Enterococcal species can cause a variety of infections, including urinary tract infections, bacteremia, endocarditis, and meningitis. Enterococci are relatively resistant to the killing effects of cell wall-active agents (penicillin, ampicillin, and vancomycin) and are impermeable to aminoglycosides.

Vancomycin-resistant enterococci (VRE) are an increasingly common and difficult-to-treat cause of hospital-acquired infection.

Multiple epidemics of VRE infection have been described in diverse hospital settings (*e.g*., medical and surgical intensive care units, and medical and pediatric wards) and, like methicillin-resistant *Staphylococcus aureus,* VRE is endemic in many large hospitals.

The factors that make these bacteria especially adept at surviving on various biomaterials include adherence and production of biofilm (see below).

The three above mentioned bacteria have the ability to form biofilms on any surface biotic and abiotic. The initial step of biofilm formation is the attachment/adherence to surface, which is stronger in shear stress conditions. The protein manly responsible for this adhesion is the polysaccharide intercellular adhesin (PIA), which allows bacteria to bind to each other, as well as to surfaces, creating the biofilm. The second stage of biofilm formation is the development of a community structure and ecosystem, which gives rise to the mature biofilm. The final stage is the detachment from the surface with consequent spreading into other locations. In all the phases of biofilm formation the quorum sensing (QS) system, mediating cell-to-cell communication, is involved.

Bacteria in the biofilm produce extracellular polymeric substances (EPS) consisting mainly of polysaccharides, nucleic acids (extracellular DNA) and proteins, that protect them from external threats, including immune system components and antimicrobials. Moreover, bacteria in the biofilm have a decreased metabolism, making them less susceptible to antibiotics; this is due to the fact that most antimicrobials require a certain degree of cellular activity in order to be effective. Another factor reinforcing such resistance is the impaired diffusion of the antibiotics throughout the biofilm because of the presence of the EPS matrix barrier.

It was also reported that in the biofilm there is higher rate of plasmid exchange increasing the chances of developing naturally occurring and antimicrobial-induced resistance.

Strategies that have been developed to eliminate biofilms target 3 different steps in the biofilm formation: inhibition of the initial stage, *i.e.* the adhesion of bacteria to surfaces; disrupting the biofilm architecture during the maturation process or step 2; inhibiting the QS system or step 3.

Because of the high resistance of these biofilms to antibiotics there is an increasing need of control and prevention of microbial growth and biofilm formation at step 2. The treatment in case of infected medical device is either a conservative treatment or the removal of the device together with a long treatment with antibiotics, but these approaches have high failure rates and elevated economical burden.

This is the reason why clinicians try to adopt a preventive approach by subministering antibiotics before implantation. Another solution could be the modification of the medical devices, *e.g*. surfaces coated with silver, which have antimicrobial property or with hydrogels as well as polyurethanes, which reduce bacterial adhesion, to mention few examples.

According to Eggiman in American Society for Microbiology Press, Washington, DC 2000. p.247, pacemakers and implantable cardioverter-defibrillators [ICDs]) can become infected, with a rate of infections ranging from 0.8 to 5.7 percent.

The infection can involve subcutaneous pocket containing the device or the subcutaneous segment of the leads. Deeper infection can also occur that involves the transvenous portion of the lead, usually with associated bacteremia and/or endovascular infection.

The device and/or pocket itself can be the source of infection, usually due to contamination at the time of implantation, or can be secondary to bacteremia from a different source.

Perioperative contamination of the pacemaker pocket with skin flora appears to be the most common source of subcutaneous infection.

Cardiac device-related infective endocarditis (CDRIE) is another life-threatening condition, with increasing incidence due to growing number of implantations (81000 pacemaker implantation per year in Europe). The incidence of CDRIE reaches 0.14 percent, and is even higher after ICD implantation.

*Staphylococcus aureus* and coagulase-negative staphylococci (often *Staphylococcus epidermidis)* cause 65 to 75 percent of generator pocket infections and up to 89 percent of device-related endocarditis. Episodes arising within two weeks of implantation are more likely to be due to *S. aureus.*

Successful treatment of an infected medical device or biomaterial, regardless of the involved component, generally requires removal of the entire system and administration of antibiotics targeting the causative bacteria. Importantly, medical therapy alone is associated with high mortality and risk of recurrence.

Prosthetic valve endocarditis (PVE) is a serious infection with potentially fatal consequences.

Bacteria can reach the valve prosthesis by direct contamination intraoperatively or via hematogenous spread during the initial days and weeks after surgery. The bacteria have direct access to the prosthesis-annulus interface and to perivalvular tissue along suture pathways because the valve sewing ring, cardiac annulus, and anchoring sutures are not endothelialized early after valve implantation. These structures are coated with host proteins, such as fibronectin and fibrinogen, to which some organisms can adhere and initiate infection.

The risk of developing prosthetic valve endocarditis (PVE) is greatest during the initial three months after surgery, remains high through the sixth month, and then falls gradually with an annual rate of approximately 0.4 percent from 12 months postoperatively onward. The percentage of patients developing PVE during the initial year after valve replacement ranges from 1 to 3 percent in studies with active follow-up; by five years, the cumulative percentage ranges from 3 to 6 percent.

The most frequently encountered pathogens in early PVE (within two months of implantation) are *S*. *aureus* and coagulase-negative staphylococci.

The most frequently encountered pathogens in late PVE (two months after valve implantation) are streptococci and *S*. *aureus,* followed by coagulase-negative staphylococci and enterococci.

The coagulase-negative staphylococci causing PVE during the initial year after surgery are almost exclusively *Staphylococcus epidermidis.* Between 84 and 87 percent of these organisms are methicillin resistant and thus resistant to all of the beta-lactam antibiotics.

According to the 2008 French survey, PVE accounts for about 20 percent of all infective endocarditis. PVE is related to health care in about 30 percent of cases. *S. aureus* is the first causative pathogen, being responsible for more than 20 percent of PVE. Importantly, when comparing data from 1999, PVE-related mortality remains high, reaching about 40 percent after surgery, and 25 percent in-hospital mortality.

Periprosthetic joint infection (PJI) occurs in 1 to 2 percent of joint replacement surgeries and is a leading cause of arthroplasty failure.

Biofilms play an important role in the pathogenesis of PJIs. Bacteria within biofilm become resistant to therapy; as a result, antibacterial therapy is often unsuccessful unless the biofilm is physically disrupted or removed by surgical debridement. Prosthetic joint infections are categorized according to the timing of symptom onset after implantation: early onset (<3 months after surgery), delayed onset (from 3 to 12 months after surgery), and late onset (>12 months after surgery). These infections have the following characteristics. Early-onset infections are usually acquired during implantation and are often due to virulent organisms, such as *Staphylococcus aureus,* or mixed infections. Delayed-onset infections are also usually acquired during implantation. Consistent with the indolent presentation, delayed infections are usually caused by less virulent bacteria, such as coagulase-negative staphylococci or enterococci. Late-onset infections resulting from hematogenous seeding are typically acute and often due to *S. aureus,* or beta hemolytic streptococci.

The management of PJIs generally consists of both surgery and antibacterial therapy.

There is therefore an urgent need in the art for a new antibacterial therapy.

WO2009/034386 describes triazolopyrimidines which are useful in the treatment of gram-positive bacterial infections, particularly *E.faecalis or E.faecium* infection but these compounds are different because the triazole nitrogen atom cannot be substituted by a cycloalkyl group. Moreover no inhibition example are reported for triazolo derivatives but for purin derivates only.

We have surprisingly found that Triazolo(4,5-d)pyrimidine derivatives possess antibacterial activity and can be used in the treatment or prevention of bacterial infection in a host mammal.

We have also found that such Triazolo(4,5-d)pyrimidine derivatives can also be used in a method for controlling bacterial growth in biofilm formation at early stage such as step 1 or 2 or for killing bacteria at all steps of biofilm formation including the latest step 3 wherein the biofilm has reached its maturation stage of matrix formation and start detachment from the surface with a consequent spreading of bacteria into other locations.

In a first aspect, the invention provides therefore Triazolo(4,5-d)pyrimidine derivatives for use in the treatment or prevention of bacterial infection in a host mammal in need of such treatment.

By bacterial infection one means for example pneumonia, septicemia, endocarditis, osteomyelitis, meningitis, urinary tract, skin, and soft tissue infections. The source of bacterial infection is diverse, and can be caused for example by the use of implantable biomaterials.

By biomaterials, one means all implantable foreign material forclinical use in host mammals such as for prosthetic joints, pacemakers, implantable cardioverter-defibrillators , intravascular catheters, coronary stent, prosthetic heart valves, intraocular lens, dental implants and the like.

By Triazolo(4,5-d)pyrimidine derivatives one means compounds of the following formula (I) 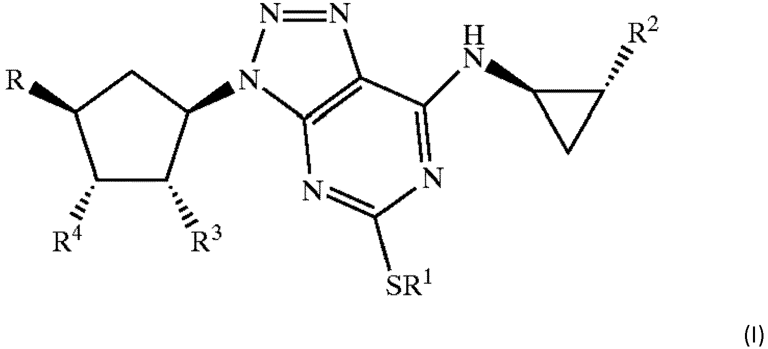
wherein R1 is C3-5 alkyl optionally substituted by one or more halogen atoms; R₂ is a phenyl group, optionally substituted by one or more halogen atoms; R₃ and R₄ are both hydroxyl; R is XOH, where X is CH₂, OCH₂CH₂, or a bond;
or a pharmaceutical acceptable salt or solvate thereof, or a solvate thereof or a solvate of such a salt provided that when X is CH₂ or a bond, R₁ is not propyl; when X is CH₂ and R₁ CH₂CH₂CF₃, butyl or pentyl, the phenyl group at R₂ must be substituted by fluorine; when X is OCH₂CH₂ and R₁ is propyl, the phenyl group at R₂ must be substituted by fluorine.

Alkyl groups whether alone or as part of another group are straight chained and fully saturated.

R₁ is a C₃₋₅ alkyl optionally substituted by one or more fluorine atoms. Preferably R₁ is 3,3,3, -trifluoropropyl, butyl or propyl.

R₂ is phenyl or phenyl substituted by one or more halogen atoms. Preferably R₂ is phenyl substituted by fluorine atoms. Most preferably R₂ is 4-fluorophenyl or 3,4-difluorophenyl.

R is is XOH, where X is CH₂, OCH₂CH₂, or a bond; preferably R is OH or OCH₂CH2OH. Most preferred Triazolo(4,5-d)pyrimidine derivatives are the ones including R2 as 4-fluorophenyl or 3,4-difluorophenyl and or R as OCH₂CH₂OH.

Triazolo(4,5-d)pyrimidine derivatives are well-known compounds. They may be obtained according to the method described in US 6,525,060. Triazolo(4,5-d)pyrimidine derivatives are used as medicament against platelet adhesion and aggregation that are preliminary steps in arterial thrombosis.

They work by antagonizing the platelet P2Y12 receptor for ADP in a reversible manner, providing rapid onset of antiplatelet effects after oral administration.P2Y12 is one of the two ADP receptors expressed by platelets, acting by amplifying platelet responses to other agonists, which stabilizes platelet aggregates and promotes thrombosis. As a consequence, P2Y12 inhibitors, alone or in combination with aspirin, significantly improve outcomes of patients with acute coronary syndromes (ACS).

We have now surprisingly found that such Triazolo(4,5-d)pyrimidine derivatives have also an antibacterial effect.

Prefered Triazolo(4,5-d)pyrimidine derivatives are derivatives with R equals OH or OCH₂CH2OH and /or R₂ equals 4-fluorophenyl or 3,4 difluorophenyl.

Most preferred Triazolo(4,5-d)pyrimidine derivatives are (1R-(1α, 2α, 3β(1R*, 2*),5β))-3-(7-((2-(3,4-difluorophenyl)cyclopropyl)amino)-5-((3,3,3-trifluoropropyl)thio)3H-1,2,3-triazolo(4,5d)pyrimidin-3-yl)5(hydroxy)cyclopentane-1,2-diol;
(1S-(1α, 2α, 3β(1R*, 2*),5β))-3-(7-((2-(3,4-difluorophenyl)cyclopropyl)amino)-5-(propylthio)(3H-1,2,3-triazolo(4,5d)pyrimidin-3-yl)5(2-hydroxyethoxy)cyclopentane-1,2-diol;
(1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol);
(1S,2S,3R,5S)-3-[7-[(1R,2S)-2-(4-fluorophenyl)cyclopropylamino]-5-(propylthio)-3H-[1,2,3]-triazolo[4,5-d]pyrimidin-3-yl]-5-(2-hydroxyethoxy)-1,2-cyclopentanediol)
and pharmaceutical acceptable salt or solvate thereof, or a solvate thereof or a solvate of such a salt.

The most preferred Triazolo(4,5-d)pyrimidine derivative is (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) as defined in formula (II) and also called Triafluocyl hereafter. 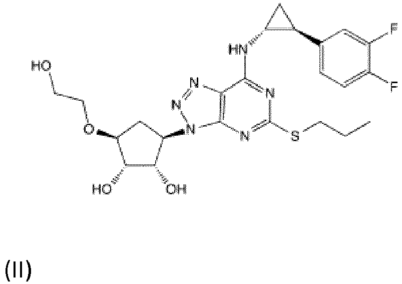 and a pharmaceutical acceptable salt or solvate thereof, or a solvate thereof or a solvate of such a salt.

According to the invention the Triazolo(4,5-d)pyrimidine derivative has to be administered to the patient over several days (especially in case of prevention).The Triazolo(4,5-d)pyrimidine derivative may be administered on their own or as a pharmaceutical composition, with non-toxic doses being inferior to 1.8 g per day.

A further preferred object of the invention is a pharmaceutical composition of Triazolo(4,5-d)pyrimidine derivative for use in the prevention or treatment of bacterial infection,

The pharmaceutical composition may be a dry powder or a liquid composition having physiological compatibility. The compositions include, in addition to triazolo(4,5-d)pyrimidine derivative, auxiliary substances, preservatives, solvents and/or viscosity modulating agents.

The Triazolo(4,5-d)pyrimidine derivative of the present invention exhibits its effects through oral, intravenous, intravascular, intramuscular, parenteral, or topical administration, and can be additionally used into a composition for parenteral administration, particularly an injection composition or in a composition for topical administration. It can also be loaded in nanoparticles for nanomedicine applications. It can be used in an aerosol composition. Such aerosol composition is for example a solution, a suspension, a micronised powder mixture and the like. The composition is administered by using a nebulizer, a metered dose inhaler or a dry powder inhaler or any device designed for such an administration.

Examples of galenic compositions include tablets, capsules, powders, pills, syrups, chewing, granules, and the like. These may be produced through well known technique and with use of typical additives such as excipients, lubricants, and binders.

Suitable auxiliary substances and pharmaceutical compositions are described in Remington's Pharmaceutical Sciences, 16th ed., 1980, Mack Publishing Co., edited by Oslo et al. Typically, an appropriate amount of a pharmaceutically-acceptable salt is used in the composition to render the composition isotonic. Examples of pharmaceutically acceptable substances include saline, Ringer's solution and dextrose solution. pH of the solution is preferably from about 5 to about 8, and more preferably from about 7 to about 7.5.

A still further preferred object of the invention is the Triazolo(4,5-d)pyrimidine derivative of formula (I) for use in a method of treatment or prevention of bacterial infection in a host mammal in need of such treatment which comprises administering to the host an effective amount of triazolo(4,5-d)pyrimidine derivatives as defined in formula (I),
preferably (1R-(1α, 2α, 3β(1R*, 2*),5β))-3-(7-((2-(3,4-difluorophenyl)cyclopropyl)amino)-5-((3,3,3-trifluoropropyl)thio)3H-1,2,3-triazolo(4,5d)pyrimidin-3-yl)5(hydroxy)cyclopentane-1,2-diol;
(1S-(1α, 2α, 3β(1R*, 2*),5β))-3-(7-((2-(3,4-difluorophenyl)cyclopropyl)amino)-5-(propylthio)(3H-1,2,3-triazolo(4,5d)pyrimidin-3-yl)5(2-hydroxyethoxy)cyclopentane-1,2-diol;
(1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol);
and most preferably (1S,2S,3R,5S)-3-[7-[(1R,2S)-2-(4-fluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) as defined in formula II;
or a pharmaceutical acceptable salt or solvate thereof, or a solvate thereof or a solvate of such a salt.

In another aspect, the invention provides the use of Triazolo(4,5-d)pyrimidine derivatives, preferably (1R-(1α, 2α, 3β(1R*, 2*),5β))-3-(7-((2-(3,4-difluorophenyl)cyclopropyl)amino)-5-((3,3,3-trifluoropropyl)thio)3H-1,2,3-triazolo(4,5d)pyrimidin-3-yl)5(hydroxy)cyclopentane-1,2-diol;
(1S-(1α, 2α, 3β(1R*, 2*),5β))-3-(7-((2-(3,4-difluorophenyl)cyclopropyl)amino)-5-(propylthio)(3H-1,2,3-triazolo(4,5d)pyrimidin-3-yl)5(2-hydroxyethoxy)cyclopentane-1,2-diol;
(1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol);
(1S,2S,3R,5S)-3-[7-[(1R,2S)-2-(4-fluorophenyl)cyclopropylamino]-5-(propylthio)-3H-[1,2,3]-triazolo[4,5-d]pyrimidin-3-yl]-5-(2-hydroxyethoxy)-1,2-cyclopentanediol)
and pharmaceutical acceptable salt or solvate thereof, or a solvate thereof or a solvate of such a salt;
and most preferably (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) or a pharmaceutical acceptable salt or solvate thereof, or a solvate thereof or a solvate of such a salt;
as inhibitor of biofilm on a surface, particularly a surface of a biomaterial or of a medical device.

By surface one means any type of surface such as plastic surface as for example surface made of polyethylene, polypropylene, polyurethane, silicone or the like but also and preferably metallic surface such as stainless steel, silver, gold, titanium, metallic alloys pyrolitic carbon, and the like. It can also be used on biomaterial surface such as biological prosthesis or devices which are made of biological material such as for example porcine or bovine pericardium

By inhibition of biofilm on a surface one means inhibition of the biofilm formation at all stages of its formation starting from a prevention or an inhibition of adherence of bacteria on the surface at step 1 but also and mainly an inhibition in bacteria grow, multiplication, and formation of microcolonies on the surface at step 2. By inhibition of biofilm one also means inhibition of the matrix at the maturation step 3 and inhibition of bacteria dispersion from the matrix in a colonisation step. By inhibition of biofilm, one also means killing bacteria at all steps of the biofilm formation.

By medical device one means biomaterial as defined above but also medical device requesting a low level of bacterial contamination such as wound dressing, soft tissue fillers, root canal fillers, contact lens, blood bag and the like.

A last further aspect according to the invention, is a method for killing or controlling bacterial growth in biofilm formation on a surface comprising applying Triazolo(4,5-d)pyrimidine derivative on a surface either at a prevention step, reducing bacteria adherence on the substrate or at a stage where the biofilm is already present, or even at a maturation step with a matrix formation wherein a more complex architecture of biofilm is established protecting bacteria as a barrier to conventional antibacterial agent.

The method of killing or controlling bacterial growth on a surface is generally applied to biomaterials or medical devices.

The biomaterial or medical device are preferably implantable foreign material for clinical use in host mammals such as prosthetic devices, pacemakers, implantable cardioverter-defibrillators, intravascular catheters, coronary stent, heart valves, intraocular lens and the like but could be extended to other medical devices requesting a low level of bacterial contamination such as for example wound dressings, soft tissue fillers containing local anaesthetics, root canal fillers with ancillary medicinal substances and the like.

The method of killing or controlling bacterial growth could also be applied to surface of experimental device in need of such antibacterial treatment.

### Description of the Figures

**Figure 1** **illustrates a Bacteriostatic and bactericidal effect of Triafluocyl on *Staphylococcus aureus.*** Growth curves (A) and viable counts (B) in the presence of different concentrations of Triafluocyl or DMSO as vehicle are shown.
**Figure 2** **illustrates an inhibition of *Staphylococcus aureus* biofilm formation by Triafluocyl at stage 2.**
**Figure 3** **illustrates a bacteriostatic and bactericidal effect of Triafluocyl on *Enterococcus faecalis.*** Growth curves (A) and viable count (B) in the presence of different concentrations of Triafluocyl or DMSO as vehicle are shown.

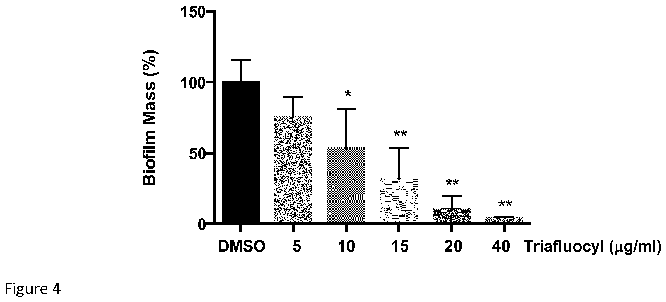

**Figure 4** **illustrates an inhibition of *Staphylococcus aureus* biofilm formation by Triafluocyl at stage 2.**
**Figure 5** **illustrates a bacteriostatic and bactericidal effect of Triafluocyl on *Staphylococcus epidermidis.*** Growth curve (upper panel) and viable count (lower panel) in the presence of different concentrations of Triafluocyl or DMSO as vehicle.
**Figure 6** **illustrated an inhibition of *Staphylococcus epidermidis* biofilm formation at stage 2 by Triafluocyl:** effect of a 24hr incubation with Triafluocyl.
**Figure 7** **illustrates a destruction of mature biofilm (stage 3: 24-hour biofilm) by Triafluocyl.** Viable count of S *epidermidis* biofilm treated after a 24h treatment with Triafluocyl (upper panel). Percentage of live cells in the biofilm (lower panel).

### Examples:

The invention is illustrated hereafter by the following non limiting examples.

We have conducted in vitro experiments, using *S*. *aureus, S. epidermidis, and E. faecalis* as a clinically relevant Gram-positive bacterial strains.

The tests were performed in accordance with the recommendations of the European Committee on Antimicrobial Susceptibility Testing **(EUCAST).**

### Example 1: use of (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) or Triafluocyl (Cayman, item No15425).

*S. aureus* (ATCC 25904) was grown overnight in Tryptic Soy Broth (TSB) medium, diluted 1:100 in fresh TSB, and incubated aerobically at 37°C until bacteria growth reached a logarithmic phase (OD₆₀₀ = 0.25-0.3).

Increasing concentrations of Triafluocyl (Cayman Chemical, Item No. 15425) or vehicle (DMSO) was then added in 5 ml of bacteria suspensions. Bacterial growth was measured after different time intervals (20-100 min) by spectrophotometry (OD₆₀₀) and by counting the colony-forming units after plating appropriate culture dilutions on TS agar plates.

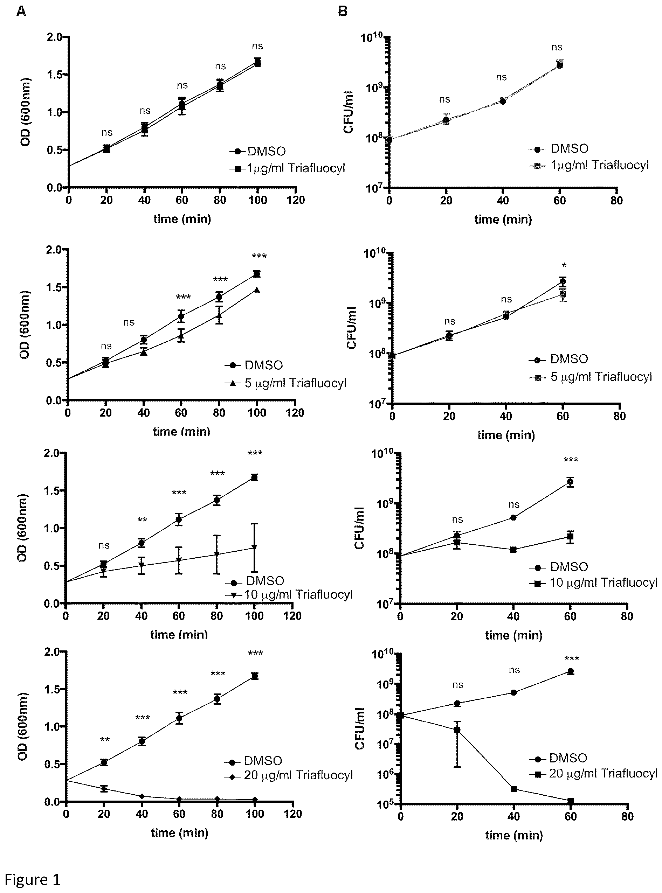

Bacteriostatic and bactericidal effects were measured with Triafluocyl. In Figure 1 kinetics of S. *aureus* growth in the presence of an increasing concentrations (1µg/ml to 20 µg/ml) of Triafluocyl were measured by turbidity measurement (upper graph), and viable count (lower graph). Data represent medians ± range (n = 3). * p<0.05; ** p<0.01, *** p<0,001, Triafluocyl vs vehicle.

As shown in Figure 1, while a concentration of 10 µg/ml Triafluocyl was able to inhibit bacterial growth, 20 µg/ml Triafluocyl displayed potent bactericidal effect.

### Example 2: use of (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) or Triafluocyl as inhibitor of biofilm formation.

*S. aureus* (ATCC 25904) was grown overnight in TSB medium, before being diluted 100 fold in fresh TSB, and incubated aerobically at 37°C until bacteria culture reached an OD₆₀₀ of 0.6 (corresponding to approximately 1-3x10⁸ CFU/ml). Bacteria cultures were then diluted to 1x10⁴ CFU/ml in fresh TSB. 800 µl aliquots of diluted bacteria suspensions were distributed in each well of a 24-well plate. Bacteria were allowed to adhere for 4 hours under static conditions at 37°C. After removing media, wells were rinsed 2 times with PBS to eliminate planktonic bacteria and re-filled with TSB supplemented with 0.5 % glucose

Triafluocyl or DMSO as vehicle was then added at desired concentration and plates were incubated at 37°C for 20 hours. After incubation, wells were washed and stained with 0.5 % (w/v) crystal violet for 30 minutes, washed again and the dye was solubilized by adding 20 % acetic acid (v/v in water) before reading absorbance at 595 nm.

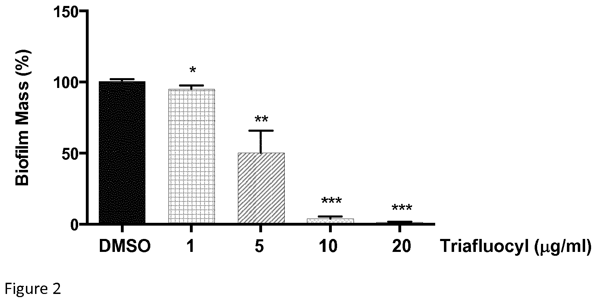

S. aureus biofilms were formed on polystyrene surface in the presence of increasing concentrations of Triafluocyl or DMSO as vehicle. In figure 2, Biofilm mass is presented as percentage of values obtained in the presence of DMSO (**P* < 0.05; ** *P* < 0.01; *** *P* < 0.001, Triafluocyl *versus* DMSO, n= 4).

Triafluocyl significantly reduces S. aureus biofilm formation at all concentrations tested. In the presence of 10 µg/ml Triafluocyl, no biofilm could form on polystyrene surface.

### Example 3: use of (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) or Triafluocyl (Cayman Chemical, Item No. 15425) .

*E. faecalis* (ATCC 29212) was grown overnight in Brain heart infusion (BHI) medium, diluted 1:100 in fresh BHI, and incubated aerobically at 37°C until bacteria growth reached a logarithmic phase (OD₆₀₀ = 0.25-0.3).

Increasing concentrations of Triafluocyl (Cayman Chemical, Item No. 15425) or DMSO as vehicle was then added in 5 ml of bacteria suspensions. Bacterial growth was measured after different time intervals (30-120 min) by spectrophotometry (OD₆₀₀) and by counting the colony-forming units after plating appropriate culture dilutions on BHI agar plates.

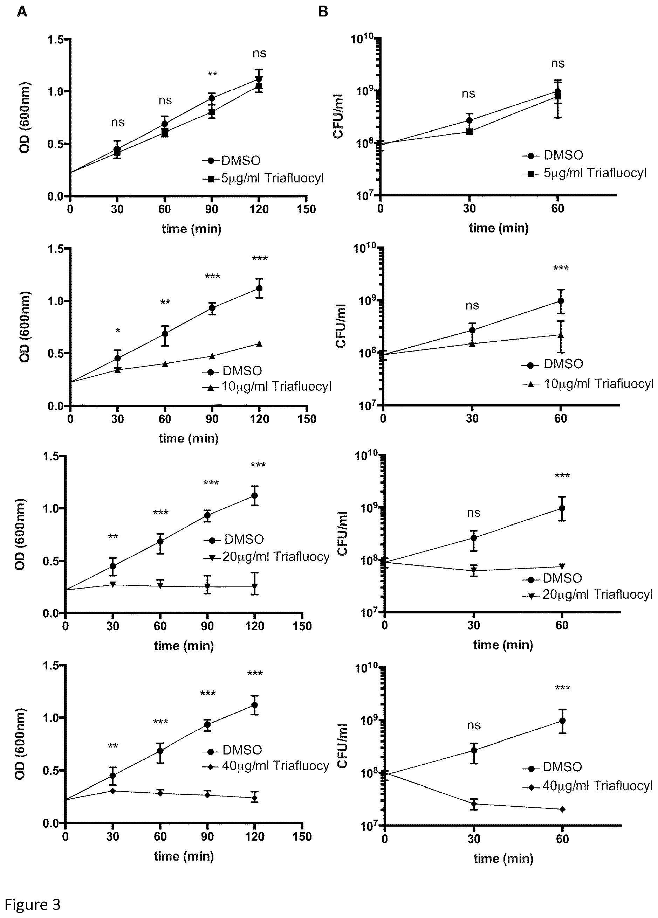

Bacteriostatic and bactericidal effects were measured with Triafluocyl. In Figure 3 kinetics of *E.faecalis* growth in the presence of an increasing concentrations (5µg/ml to 40 µg/ml) of Triafluocyl were measured by turbidity measurement (upper graph), and viable count (lower graph). Data represent medians ± range (n = 3).

As shown in Figure 3, while a concentration of 10 µg/ml Triafluocyl was able to inhibit bacterial growth, 20 µg/ml Triafluocyl and more importantly 40 µg /ml displayed potent bactericidal effects.

### Example 4: use of Triafluocyl as inhibitor of biofilm formation.

*E. faecalis* (ATCC 29212) was grown overnight in BHI medium, before being diluted 100 fold in fresh TSB, and incubated aerobically at 37°C until bacteria culture reached an OD₆₀₀ of 0.6 (corresponding to approximately 2-5x10⁸ CFU/ml). Bacteria cultures were then diluted to 1x10⁴ CFU/ml in fresh TSB. 800 µl aliquots of diluted bacteria suspensions were distributed in each well of a 24-well plate. Bacteria were allowed to adhere for 4 hours under static conditions at 37°C. After removing media, wells were rinsed 2 times with PBS to eliminate planktonic bacteria and re-filled with TSB supplemented with 0.5 % glucose

Triafluocyl or DMSO as vehicle was then added at desired concentration and plates were incubated at 37°C for 20 hours. After incubation, wells were washed and stained with 0.5 % (w/v) crystal violet for 30 minutes, washed again and the dye was solubilized by adding 20 % acetic acid (v/v in water) before reading absorbance at 595 nm.

*E.faecalis* biofilms were formed on polystyrene surface in the presence of increasing concentrations of Triafluocyl or DMSO as vehicle. In figure 2, Biofilm mass is presented as percentage of values obtained in the presence of DMSO (**P* < 0.05; ** *P* < 0.01; *** *P* < 0.001, Triafluocyl *versus* DMSO, n= 4).

Triafluocyl significantly reduces *E.faecalis* biofilm formation at a starting concentration of 10 µg/ml. In the presence of 40 µg/ml Triafluocyl, no biofilm could form on polystyrene surface.

### Example 5: Triafluocyl effect on S. epidermidis: Susceptibility testing: MIC and MBC determination.

The Minimal Inhibitory Concentration (MIC) and the Minimal Bactericidal Concentration (MBC) of Triafluocyl were determined in *Staphylococcus epidermidis* (ATCC 35984, also known as RP62A) according to EUCAST recommendations.

Briefly, a single colony grown on a Tryptic Soy Agar (TSA) plate was resuspended and cultured in Tryptic Soy Broth (TSB) overnight (O/N) in aerobic conditions (37°C with 220rpm shaking), next day a 1:50 inoculum in Mueller-Hinton broth (MHB) was incubated in aerobic conditions for 3hr and an inoculum of 1:100 dilution, corresponding to 3x10⁵ CFU/ml, was incubated in presence or absence of different concentrations of Triafluocyl in 1% DMSO (vehicle). After O/N growth the OD of each culture was measured at 600nm in a spectrophotometer (OD₆₀₀). The MIC represents the concentration at which there is no visible growth of bacteria, *i.e.* ΔOD at 600nm equal to zero (blank is the medium alone).

We have also determined the MBC, *i.e.* the concentration at which the liquid culture, when spread on TSA plates, will not produce any colony.

The MIC for Triafluocyl against *S.epidermidis* is equal to 12 ± 3 µg/ml and the MBC is 17 ± 3 µg/ml (two biological replicates, detection limit 10⁻³).

### Example 6: Time-kill study of S. epidermidis in logarithmic phase

To evaluate Triafluocyl antibacterial effect we have tested *S*. *epidermidis* liquid growth in the presence of different Triafluocyl concentrations in logarithmic phase. In this phase usually bacteria are highly susceptible to agents with bactericidal activity because they are rapidly dividing.

A 1:100 inoculum in 50ml TSB of an O/N culture of *S*. *epidermidis* was cultured for 3hr up to its logarithmic phase (OD₆₀₀ = 0,26 and ≈ 3x10⁸ CFU/ml).

Bacteria were split in several tubes containing different concentrations of DMSO as vehicle alone or in combination with Triafluocyl in TSB and grown for 100min at 37°C with 220rpm shaking, the OD₆₀₀ was measured every 20min.

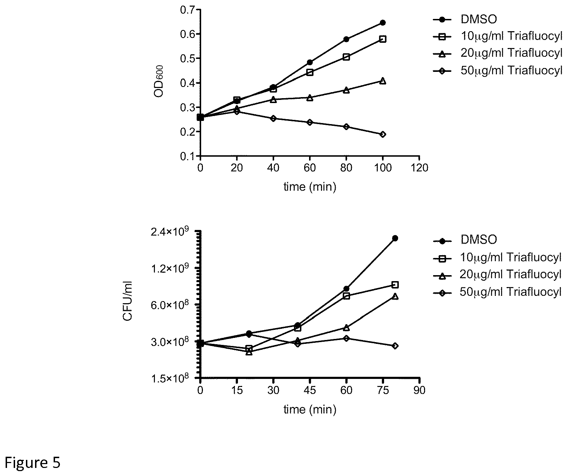

Compared to the growth with DMSO (0,25%) we observed a dose-dependent inhibition of *S*. *epidermidis* growth between 10µg/ml and 20µg/ml Triafluocyl (Figure 5). At 50µg/ml we observed a slight bacteriostatic activity, confirmed by the number of viable cells at 80min, 3x10⁸ CFU/ml, equal to the number of bacteria in the untreated control at the beginning of the assay (Figure 5).

Moreover, we have tested the effect of Triafluocyl on a low-density inoculum, 0.08x10⁶ CFU/ml, from a culture of *S*. *epidermidis* in logarithmic phase. We have followed the growth for 4hr with or without Triafluocyl and measured the OD₆₀₀: already 5µg/ml of Triafluocyl decreased the OD by 50% compared to the growth in absence of Triafluocyl at the same time point; 10µg/ml and 20µg/ml inhibited growth (OD value equal to OD at the beginning of the growth) (data not shown).

This means that the lower the inoculum density the lower the concentration of Triafluocyl to slow down growth or kill bacteria.

### Example 7: Triafluocyl affects S. epidermidis biofilm formation

To study the effect of Triafluocyl on biofilm formation, *S*. *epidermidis* in early logarithmic phase (5x10⁸ CFU/ml) was plated in a 24-well plate and let to adhere at the bottom of the well for 4hr at 37°C in static conditions. After 4hr incubation, planktonic bacteria were removed and adherent bacteria were washed twice in TSB. Fresh TSB medium supplemented or not with 0.25% glucose was added to the well with 5 different concentrations of Triafluocyl and incubated for 24 hours. Wells were washed 3 times with NaCl 0.9% and incubated for 1hr at RT with Crystal Violet 1% solution in dH₂O to stain the biofilm.

Wells were washed 3 times with dH₂O to eliminate unbound crystal violet, then 400µl Acetic Acid 10% was added and incubated at RT for 10min. Absorbance was measured in triplicate at 570nm, reflecting total biomass of the biofilm (live and dead bacteria).

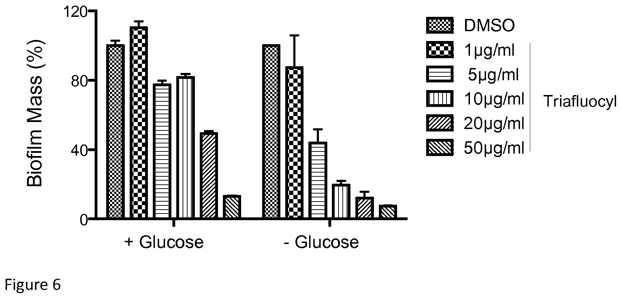

Triafluocyl affected biofilm formation (Figure 6): already at at 5µg/ml, in the absence of glucose, it inhibited biofilm formation by 50%, while in presence of glucose we reach 50% biofilm reduction only at 20µg/ml Triafluocyl.

The concentration of Triafluocyl that inhibits at least 90% biofilm formation is called minimum biofilm inhibitory concentration (MBIC). Triafluocyl MBIC for *S. epidermidis* is 50µg/ml both in the presence and in the absence of glucose.

### Example 8: Triafluocyl destroys S. epidermidis mature biofilm

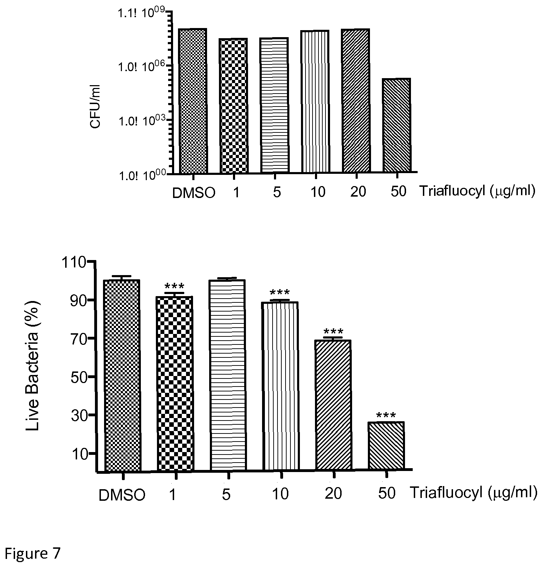

In another experiment we let adhere 0.5x10⁸ CFU/ml *S*. *epidermidis* cells for 4hr and let the biofilm form for additional 24hr in presence of 0.25% glucose, at this point we treated the biofilm with several concentrations of Triafluocyl for 24hr in TSB with 0.25% glucose and determined the viable count (Figure 7) as well as the percentage of live cells using the BacLight bacterial viability kit (Molecular Probes).

For biofilm analysis, we first washed the biofilm to eliminate all planktonic bacteria and then the biofilm was detached mechanically using a scraper. To assure that the aggregates from the biofilm were completely dissociated, the suspension of cells was passed through a needle (0.5x16mm) and a dilution was plated on TSA plates.

Only the highest concentration of Triafluocyl, 50µg/ml, was effective in reducing the number of viable cells in the biofilm with a reduction of almost 3 log (from 1.1x10⁸ CFU/ml in the control to 1.5x10⁵ CFU/ml).

In the same experiment we also determined the percentage of live and dead bacteria. To do so we followed the procedure of the kit LIVE/DEAD from Molecular Probes. Briefly, the biofilm was resuspended in a solution of 0.9% NaCl and cells were stained with a mixture of SYTO9 (green fluorescence) and propidium iodide (PI) (red fluorescence) for 15 min in the dark. Stained cells were transferred in a 96-well plate and fluorescence was measured using the Enspire Spectrophotometer with excitation wavelength of 470nm and emission spectra in the range of 490 - 700nm. SYTO9 dye (green fluorescence 500-520nm) penetrates all the cells (dead and live) and binds to DNA, while PI (red fluorescence in the range 610-630nm) enters only in dead cells with a damaged cell membrane. When PI and SYTO9 are in the same cell the green fluorescence intensity decreases. Therefore, in a population of cells with high percentage of dead cells there is a reduction in the emission spectra of the green fluorescence, because there is more PI staining. The ratio of fluorescence intensity (green/live) is plotted against a known percentage of live cells to obtain a standard curve and the percentage of live cells in our samples is obtained by extrapolation (Figure 7). Triafluocyl, at concentrations of 20µg/ml and 50µg/ml reduced the percentage of live bacteria to 80% and 30%, respectively.

## Claims

1. A Triazolo(4,5-d)pyrimidine derivative of formula (I) 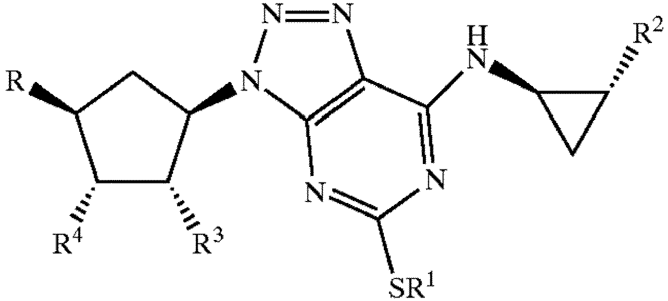
wherein R₁ is C₃₋₅ alkyl optionally substituted by one or more halogen atoms; R₂ is a phenyl group, optionally substituted by one or more halogen atoms; R₃ and R₄ are both hydroxyl; R is XOH, where X is CH₂, OCH₂CH₂, or a bond;
or a pharmaceutical acceptable salt or solvate thereof, or a solvate of such a salt provided that when X is CH₂ or a bond, R₁ is not propyl; when X is CH₂ and
R₁ CH₂CH₂CF₃, butyl or pentyl, the phenyl group at R₂ must be substituted by fluorine; when X is OCH₂CH₂ and R₁ is propyl, the phenyl group at R₂ must be substituted by fluorine;
for use in treatment or prevention of bacterial infection in a host mammal in need of such treatment.

2. Use of a Triazolo(4,5-d)pyrimidine derivative of formula (I) as inhibitor of Gram-positive biofilm formation on a surface, wherein the use is not a method of treatment of the human or animal body by therapy, 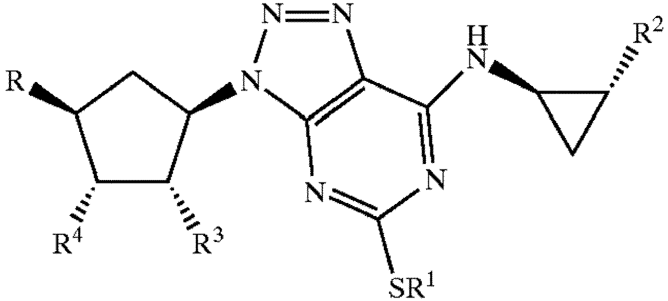
wherein R₁ is C₃₋₅ alkyl optionally substituted by one or more halogen atoms; R₂ is a phenyl group, optionally subs;tituted by one or more halogen atoms; R₃ and R₄ are both hydroxyl; R is XOH, where X is CH₂, OCH₂CH₂, or a bond;
or a pharmaceutical acceptable salt or solvate thereof, or a solvate of such a salt provided that when X is CH₂ or a bond, R₁ is not propyl; when X is CH₂ and R₁ CH₂CH₂CF₃, butyl or pentyl, the phenyl group at R₂ must be substituted by fluorine; when X is OCH₂CH₂ and R₁ is propyl, the phenyl group at R₂ must be substituted by fluorine;

3. The triazolo(4,5-d) pyrimidine derivative for use according to claim 1 wherein R₂ is phenyl substituted by fluorine atoms.

4. The triazolo(4,5-d) pyrimidine derivative for use according to any one of claim 1 or 3 wherein R is OH or OCH₂CH₂OH.

5. The triazolo(4,5-d) pyrimidine derivative for use according to claim 4 selected from:
(1R-(1α, 2α, 3β(1R*, 2*),5β))-3-(7-((2-(3,4-difluorophenyl)cyclopropyl)amino)-5-((3,3,3-trifluoropropyl)thio)3H-1,2,3-triazolo(4,5d)pyrimidin-3-yl)5(hydroxy)cyclopentane-1,2-diol;
(1S-(1α, 2α, 3β(1R*, 2*),5β))-3-(7-((2-(3,4-difluorophenyl)cyclopropyl)amino)-5-(propylthio)(3H-1,2,3-triazolo(4,5d)pyrimidin-3-yl)5(2-hydroxyethoxy)cyclopentane-1,2-diol;
(1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol);
(1S,2S,3R,5S)-3-[7-[(1R,2S)-2-(4-fluorophenyl)cyclopropylamino]-5-(propylthio)-3H-[1,2,3]-triazolo[4,5-d]pyrimidin-3-yl]-5-(2-hydroxyethoxy)-1,2-cyclopentanediol);
and pharmaceutical acceptable salt or solvate thereof, or a solvate thereof or a solvate of such a salt.

6. The Triazolo(4,5-d)pyrimidine derivative for use according to any one of claim 1 and 3 to 5 which is (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) also called Triafluocyl.

7. The Triazolo(4,5-d)pyrimidine derivative of formula (I) for use according to claim 1 in a method for treatment of a Gram-positive bacterial infection comprising administering to the host, the Triazolo(4,5-d)pyrimidine derivative in an effective amount inferior to 1.8 g per day .

8. Triafluocyl for use according to claim 6 in a method for treatment of a Gram-positive bacterial infection comprising administering to the host, the Triafluocyl in an effective amount inferior to 1.8 g per day

9. Use of a Triazolo(4,5-d)pyrimidine derivative of formula (I) as inhibitor of biofilm formation on a surface according to claim 2 wherein R₂ is phenyl substituted by fluorine atoms.

10. Use of a Triazolo(4,5-d)pyrimidine derivative of formula (I) as inhibitor of biofilm formation on a surface according to claim 2 and 9 wherein R is OH or OCH₂CH₂OH.

11. Use of a Triazolo(4,5-d)pyrimidine derivative of formula (I) as inhibitor of biofilm formation on a surface according to claim 10 wherein the Triazolo(4,5-d) pyrimidine derivative is selected from:
(1R-(1α, 2α, 3β(1R*, 2*),5β))-3-(7-((2-(3,4-difluorophenyl)cyclopropyl)amino)-5-((3,3,3-trifluoropropyl)thio)3H-1,2,3-triazolo(4,5d)pyrimidin-3-yl) 5(hydroxy)cyclopentane-1,2-diol;
(1S-(1α, 2α, 3β(1R*, 2*),5β))-3-(7-((2-(3,4-difluorophenyl)cyclopropyl)amino)-5-(propylthio)(3H-1,2,3-triazolo(4,5d)pyrimidin-3-yl)5(2-hydroxyethoxy)cyclopentane-1,2-diol;
(1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol);
(1S,2S,3R,5S)-3-[7-[(1R,2S)-2-(4-fluorophenyl)cyclopropylamino]-5-(propylthio)-3H-[1,2,3]-triazolo[4,5-d]pyrimidin-3-yl]-5-(2-hydroxyethoxy)-1,2-cyclopentanediol);
and pharmaceutical acceptable salt or solvate thereof, or a solvate thereof or a solvate of such a salt.

12. Use of a Triazolo(4,5-d)pyrimidine derivative of formula (I) as inhibitor of biofilm formation on a surface according to claim 10 or 11 wherein the Triazolo(4,5-d)pyrimidine derivative is (1S,2S,3R,5S)-3-[7-[(1R,2S)-2-(3,4-difluorophenyl)cyclopropylamino]-5-(propylthio)-3H-[1,2,3]-triazolo[4,5-d]pyrimidin-3-yl]-5-(2-hydroxyethoxy)-1,2-cyclopentanediol) also called Triafluocyl.

13. A method for killing or controlling Gram-positive bacterial growth in biofilm formation comprising applying on a surface, an effective amount of Triazolo(4,5-d)pyrimidine derivative of formula(I) according to claim 2.

14. The method for killing bacteria in biofilm formation on a surface according to claim 13 wherein the biofilm formation is at a maturation step 3 comprising more than 90x10⁶ CFU/cm².

15. The method according to anyone of claim 2 and 9 to 14 wherein the surface belongs to a biomaterial.

16. The method according to claim 15 wherein the biomaterial is a cardiovascular device.

17. The method according to claim 16 wherein the cardiovascular device is a prosthetic heart valve.

18. The method according to claim 16 wherein the device is a pacemaker.

## Patentansprüche

1. Triazolo(4,5-d)pyrimidinderivat der Formel (I), 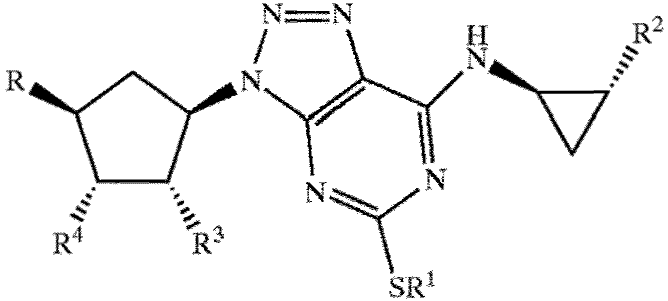
wobei R₁ C₃₋₅-Alkyl, wahlweise substituiert durch ein oder mehrere Halogenatome, ist; R₂ eine Phenylgruppe, wahlweise substituiert durch ein oder mehrere Halogenatome, ist; R₃ und R₄ beide Hydroxyl sind; R XOH ist, wobei X CH₂, OCH₂CH₂, oder eine Bindung ist;
oder ein pharmazeutisch annehmbares Salz oder Solvat davon, oder ein Solvat von solch einem Salz, mit der Maßgabe, dass, wenn X CH₂ oder eine Bindung ist, R₁ nicht Propyl ist; wenn X CH₂ ist und R₁ CH₂CH₂CF₃, Butyl oder Pentyl ist, die Phenylgruppe an R₂ durch Fluor substituiert sein muss; wenn X OCH₂CH₂ ist und R₁ Propyl ist, die Phenylgruppe an R₂ durch Fluor substituiert sein muss;
zur Verwendung bei der Behandlung oder Vorbeugung bakterieller Infektionen bei einem Wirtssäugetier, das einer solchen Behandlung bedarf.

2. Verwendung eines Triazolo(4,5-d)pyrimidinderivats der Formel (I) als Hemmer der Bildung eines grampositiven Biofilms auf einer Oberfläche, wobei die Verwendung kein Verfahren der Behandlung des menschlichen oder tierischen Körpers durch Therapie ist, 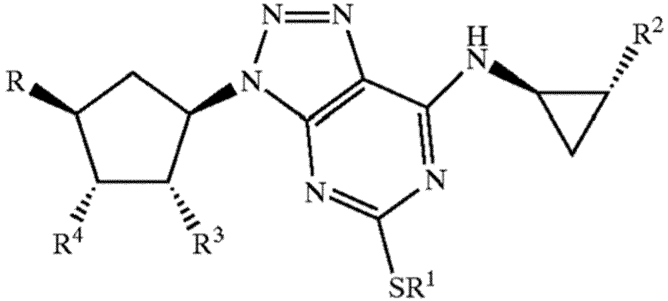
wobei R₁ C₃₋₅-Alkyl, wahlweise substituiert durch ein oder mehrere Halogenatome, ist; R₂ eine Phenylgruppe, wahlweise substituiert durch ein oder mehrere Halogenatome, ist; R₃ und R₄ beide Hydroxyl sind; R XOH ist, wobei X CH₂, OCH₂CH₂, oder eine Bindung ist;
oder ein pharmazeutisch annehmbares Salz oder Solvat davon, oder ein Solvat von solch einem Salz, mit der Maßgabe, dass, wenn X CH₂ oder eine Bindung ist, R₁ nicht Propyl ist; wenn X CH₂ ist und R₁ CH₂CH₂CF₃, Butyl oder Pentyl ist, die Phenylgruppe an R₂ durch Fluor substituiert sein muss; wenn X OCH₂CH₂ ist und R₁ Propyl ist, die Phenylgruppe an R₂ durch Fluor substituiert sein muss.

3. Triazolo(4,5-d)pyrimidinderivat zur Verwendung nach Anspruch 1, wobei R₂ Phenyl substituiert durch Fluoratome ist.

4. Triazolo(4,5-d)pyrimidinderivat zur Verwendung nach einem der Ansprüche 1 oder 3, wobei R OH oder OCH₂CH₂OH ist.

5. Triazolo(4,5-d)pyrimidinderivat zur Verwendung nach Anspruch 4, ausgewählt aus:
(1R-(1α,2α,3β(1R*,2*),5β))-3-(7-((2-(3,4-Difluorphenyl)cyclopropyl)amino)-5-((3,3,3-trifluorpropyl)thio)3H-1,2,3-triazolo(4,5d)pyrimidin-3-yl)5(hydroxy)cyclopentan-1,2-diol;
(1S-(1α,2α,3β(1R*,2*),5β))-3-(7-((2-(3,4-Difluorphenyl)cyclopropyl)amino)-5-(propylthio)(3H-1,2,3-triazolo(4,5d)pyrimidin-3-yl)5(2-hydroxyethoxy)cyclopentan-1,2-diol;
(1S,2S,3R,5S)-3-[7-[(1R,2S)-2-(3,4-Difluorphenyl)cyclopropylamino]-5-(propylthio)-3H-[1,2,3]-triazolo[4,5-d]pyrimidin-3-yl]-5-(2-hydroxyethoxy)-1,2-cyclopentandiol);
(1S,2S,3R,5S)-3-[7-[(1R,2S)-2-(4-Fluorphenyl)cyclopropylamino]-5-(propylthio)-3H-[1,2,3]-triazolo[4,5-d]pyrimidin-3-yl]-5-(2-hydroxyethoxy)-1,2-cyclopentandiol);
und einem pharmazeutisch annehmbaren Salz oder Solvat davon, oder einem Solvat davon oder einem Solvat eines solchen Salzes.

6. Triazolo(4,5-d)pyrimidinderivat zur Verwendung nach einem der Ansprüche 1 und 3 bis 5, wobei es sich um (1S,2S,3R,5S)-3-[7-[(1R,2S)-2-(3,4-Difluorphenyl)cyclopropylamino]-5-(propylthio)-3H-[1,2,3]-triazolo[4,5-d]pyrimidin-3-yl]-5-(2-hydroxyethoxy)-1,2-cyclopentandiol), auch als Triafluocyl bezeichnet, handelt.

7. Triazolo(4,5-d)pyrimidinderivat der Formel (I) zur Verwendung nach Anspruch 1, in einem Verfahren zur Behandlung einer grampositiven bakteriellen Infektion, umfassend die Verabreichung des Triazolo(4,5-d)pyrimidinderivats an den Wirt in einer wirksamen Menge von weniger als 1,8 g pro Tag.

8. Triafluocyl zur Verwendung nach Anspruch 6 in einem Verfahren zur Behandlung einer grampositiven bakteriellen Infektion, umfassend die Verabreichung des Triafluocyls an den Wirt in einer wirksamen Menge von weniger als 1,8 g pro Tag.

9. Verwendung eines Triazolo(4,5-d)pyrimidinderivats der Formel (I) als Hemmer der Biofilmbildung auf einer Oberfläche nach Anspruch 2, wobei R₂ Phenyl substituiert durch Fluoratome ist.

10. Verwendung eines Triazolo(4,5-d)pyrimidinderivats der Formel (I) als Hemmer der Biofilmbildung auf einer Oberfläche nach Anspruch 2 und 9, wobei R OH oder OCH₂CH₂OH ist.

11. Verwendung eines Triazolo(4,5-d)pyrimidinderivats der Formel (I) als Hemmer der Biofilmbildung auf einer Oberfläche nach Anspruch 10, wobei das Triazolo(4,5-d)pyrimidinderivat ausgewählt ist aus:
(1R-(1α,2α,3β(1R*,2*),5β))-3-(7-((2-(3,4-Difluorphenyl)cyclopropyl)amino)-5-((3,3,3-trifluorpropyl)thio)3H-1,2,3-triazolo(4,5d)pyrimidin-3-yl)5(hydroxy)cyclopentan-1,2-diol;
(1S-(1α,2α,3β(1R*,2*),5β))-3-(7-((2-(3,4-Difluorphenyl)cyclopropyl)amino)-5-(propylthio)(3H-1,2,3-triazolo(4,5d)pyrimidin-3-yl)5(2-hydroxyethoxy)cyclopentan-1,2-diol;
(1S,2S,3R,5S)-3-[7-[(1R,2S)-2-(3,4-Difluorphenyl)cyclopropylamino]-5-(propylthio)-3H-[1,2,3]-triazolo[4,5-d]pyrimidin-3-yl]-5-(2-hydroxyethoxy)-1,2-cyclopentandiol);
(1S,2S,3R,5S)-3-[7-[(1R,2S)-2-(4-Fluorphenyl)cyclopropylamino]-5-(propylthio)-3H-[1,2,3]-triazolo[4,5-d]pyrimidin-3-yl]-5-(2-hydroxyethoxy)-1,2-cyclopentandiol);
und einem pharmazeutisch annehmbaren Salz oder Solvat davon, oder einem Solvat davon oder einem Solvat eines solchen Salzes.

12. Verwendung eines Triazolo(4,5-d)pyrimidinderivats der Formel (I) als Hemmer der Biofilmbildung auf einer Oberfläche nach Anspruch 10 oder 11, wobei es sich bei dem Triazolo(4,5-d)pyrimidinderivat um (1S,2S,3R,5S)-3-[7-[(1R,2S)-2-(3,4-Difluorphenyl)cyclopropylamino]-5-(propylthio)-3H-[1,2,3]-triazolo[4,5-d]pyrimidin-3-yl]-5-(2-hydroxyethoxy)-1,2-cyclopentandiol), auch als Triafluocyl bezeichnet, handelt.

13. Verfahren zum Abtöten oder Regulieren des Wachstums grampositiver Bakterien bei der Biofilmbildung, umfassend das Auftragen auf einer Oberfläche einer wirksamen Menge eines Triazolo(4,5-d)pyrimidinderivats der Formel (I) nach Anspruch 2.

14. Verfahren zum Abtöten von Bakterien bei der Biofilmbildung auf einer Oberfläche nach Anspruch 13, wobei die Biofilmbildung in einem Reifungsschritt 3, umfassend mehr als 90 x 10⁶ KBE/cm², stattfindet.

15. Verfahren nach einem der Ansprüche 2 und 9 bis 14, wobei die Oberfläche zu einem Biomaterial gehört.

16. Verfahren nach Anspruch 15, wobei es sich bei dem Biomaterial um ein Herz-Kreislauf-Gerät handelt.

17. Verfahren nach Anspruch 16, wobei es sich bei dem Herz-Kreislauf-Gerät um eine Herzklappenprothese handelt.

18. Verfahren nach Anspruch 16, wobei es sich bei dem Gerät um einen Herzschrittmacher handelt.

## Revendications

1. Dérivé de triazolo(4,5-d)pyrimidine de formule (I) 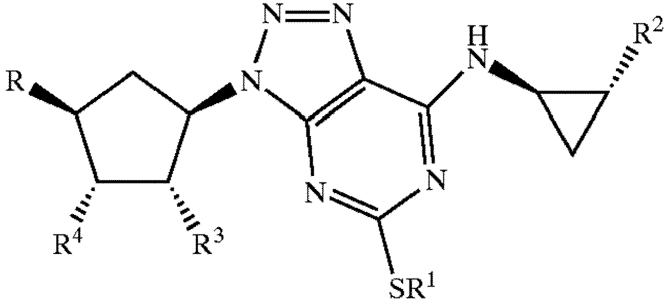
où R₁ représente un groupement alkyle en C₃₋₅ éventuellement substitué par un ou plusieurs atomes d'halogène ; R₂ représente un groupement phényle, éventuellement substitué par un ou plusieurs atomes d'halogène ; R₃ et R₄ représentent tous deux des groupements hydroxyle ; R représente XOH, où X représente CH₂, OCH₂CH₂ ou une liaison ;
ou sel pharmaceutiquement acceptable ou solvate de celui-ci, ou solvate d'un tel sel, à la condition que lorsque X représente CH₂ ou une liaison, R₁ ne représente pas un groupement propyle ; lorsque X représente CH₂ et R₁ CH₂CH₂CF₃, butyle ou pentyle, le groupement phényle de R₂ doit être substitué par fluor ; lorsque X représente OCH₂CH₂ et R₁ représente un groupement propyle, le groupement phényle de R₂ doit être substitué par fluor ;
pour utilisation dans le traitement prophylactique ou thérapeutique d'une infection bactérienne chez un mammifère hôte nécessitant un tel traitement.

2. Utilisation d'un dérivé de triazolo(4,5-d)pyrimidine de formule (I) en tant qu'inhibiteur de formation de biofilm à Gram positif sur une surface, où l'utilisation n'est pas une méthode de traitement de l'organisme humain ou animal par thérapie, 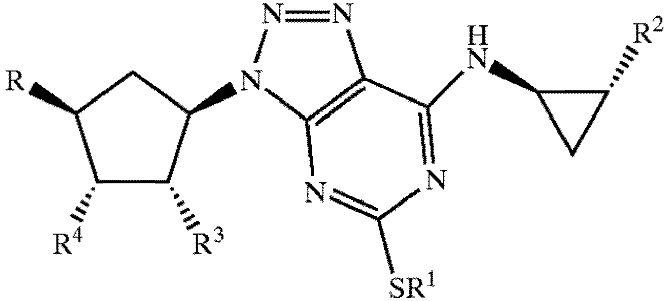
où R₁ représente un groupement alkyle en C₃₋₅ éventuellement substitué par un ou plusieurs atomes d'halogène ; R₂ représente un groupement phényle, éventuellement substitué par un ou plusieurs atomes d'halogène ; R₃ et R₄ représentent tous deux des groupements hydroxyle ; R représente XOH, où X représente CH₂, OCH₂CH₂ ou une liaison ;
ou sel pharmaceutiquement acceptable ou solvate de celui-ci, ou solvate d'un tel sel, à la condition que lorsque X représente CH₂ ou une liaison, R₁ ne représente pas un groupement propyle ; lorsque X représente CH₂ et R₁ CH₂CH₂CF₃, butyle ou pentyle, le groupement phényle de R₂ doit être substitué par fluor ; lorsque X représente OCH₂CH₂ et R₁ représente un groupement propyle, le groupement phényle de R₂ doit être substitué par fluor.

3. Dérivé de triazolo(4,5-d)pyrimidine pour utilisation selon la revendication 1, où R₂ représente un groupement phényle substitué par des atomes de fluor.

4. Dérivé de triazolo(4,5-d)pyrimidine pour utilisation selon l'une quelconque des revendications 1 ou 3, où R représente OH ou OCH₂CH₂OH.

5. Dérivé de triazolo(4,5-d)pyrimidine pour utilisation selon la revendication 4 choisi parmi :
(1R-(1α, 2α, 3β(1R*, 2*),5β))-3-(7-((2-(3,4-difluorophényl)cyclopropyl)amino)-5-((3,3,3-trifluoropropyl)thio)3H-1,2,3-triazolo(4,5d)pyrimidin-3-yl)5(hydroxy)cyclopentane-1,2-diol ;
(1S-(1α, 2α, 3β(1R*, 2*),5β))-3-(7-((2-(3,4-difluorophényl)cyclopropyl)amino)-5-(propylthio)(3H-1,2,3-triazolo(4,5d)pyrimidin-3-yl)5(2-hydroxyéthoxy)cyclopentane-1,2-diol ;
(1S,2S,3R,5S)-3-[7-[(1R,2S)-2-(3,4-difluorophényl)cyclopropylamino]-5-(propylthio)-3H-[1,2,3]-triazolo[4,5-d]pyrimidin-3-yl]-5-(2-hydroxyéthoxy)-1,2-cyclopentanediol) ;
(1S,2S,3R,5S)-3-[7-[(1R,2S)-2-(4-fluorophényl)cyclopropylamino]-5-(propylthio)-3H-[1,2,3]-triazolo[4,5-d]pyrimidin-3-yl]-5-(2-hydroxyéthoxy)-1,2-cyclopentanediol) ;
et sel pharmaceutiquement acceptable ou solvate de celui-ci, ou solvate de celui-ci ou solvate d'un tel sel.

6. Dérivé de triazolo(4,5-d)pyrimidine pour utilisation selon l'une quelconque des revendications 1 et 3 à 5 qui est le (1S,2S,3R,5S)-3-[7-[(1R,2S)-2-(3,4-difluorophényl)cyclopropylamino]-5-(propylthio)-3H-[1,2,3]-triazolo[4,5-d]pyrimidin-3-yl]-5-(2-hydroxyéthoxy)-1,2-cyclopentanediol) également appelé Triafluocyl.

7. Dérivé de triazolo(4,5-d)pyrimidine de formule (I) pour utilisation selon la revendication 1 dans une méthode de traitement d'une infection bactérienne à Gram positif comprenant l'administration à l'hôte du dérivé de triazolo(4,5-d)pyrimidine à une teneur active inférieure à 1,8 g par jour.

8. Triafluocyl pour utilisation selon la revendication 6 dans une méthode de traitement d'une infection bactérienne à Gram positif comprenant l'administration à l'hôte du Triafluocyl à une teneur active inférieure à 1,8 g par jour.

9. Utilisation d'un dérivé de triazolo(4,5-d)pyrimidine de formule (I) en tant qu'inhibiteur de formation de biofilm sur une surface selon la revendication 2, où R₂ représente un groupement phényle substitué par des atomes de fluor.

10. Utilisation d'un dérivé de triazolo(4,5-d)pyrimidine de formule (I) en tant qu'inhibiteur de formation de biofilm sur une surface selon les revendications 2 et 9, où R représente OH ou OCH₂CH₂OH.

11. Utilisation d'un dérivé de triazolo(4,5-d)pyrimidine de formule (I) en tant qu'inhibiteur de formation de biofilm sur une surface selon la revendication 10, où le dérivé de triazolo(4,5-d)pyrimidine est choisi parmi :
(1R-(1α, 2α, 3β(1R*, 2*),5β))-3-(7-((2-(3,4-difluorophényl)cyclopropyl)amino)-5-((3,3,3-trifluoropropyl)thio)3H-1,2,3-triazolo(4,5d)pyrimidin-3-yl)5(hydroxy)cyclopentane-1,2-diol ;
(1S-(1α, 2α, 3β(1R*, 2*),5β))-3-(7-((2-(3,4-difluorophényl)cyclopropyl)amino)-5-(propylthio)(3H-1,2,3-triazolo(4,5d)pyrimidin-3-yl)5(2-hydroxyéthoxy)cyclopentane-1,2-diol ;
(1S,2S,3R,5S)-3-[7-[(1R,2S)-2-(3,4-difluorophényl)cyclopropylamino]-5-(propylthio)-3H-[1,2,3]-triazolo[4,5-d]pyrimidin-3-yl]-5-(2-hydroxyéthoxy)-1,2-cyclopentanediol) ;
(1S,2S,3R,5S)-3-[7-[(1R,2S)-2-(4-fluorophényl)cyclopropylamino]-5-(propylthio)-3H-[1,2,3]-triazolo[4,5-d]pyrimidin-3-yl]-5-(2-hydroxyéthoxy)-1,2-cyclopentanediol) ;
et sel pharmaceutiquement acceptable ou solvate de celui-ci, ou solvate de celui-ci ou solvate d'un tel sel.

12. Utilisation d'un dérivé de triazolo(4,5-d)pyrimidine de formule (I) en tant qu'inhibiteur de formation de biofilm sur une surface selon la revendication 10 ou 11 où le dérivé de triazolo(4,5-d)pyrimidine est le (1S,2S,3R,5S)-3-[7-[(1R,2S)-2-(3,4-difluorophényl)cyclopropylamino]-5-(propylthio)-3H-[1,2,3]-triazolo[4,5-d]pyrimidin-3-yl]-5-(2-hydroxyéthoxy)-1,2-cyclopentanediol) également appelé Triafluocyle.

13. Méthode d'élimination ou de régulation de la croissance de bactéries à Gram positif dans la formation de biofilm comprenant l'application sur une surface d'une quantité active d'un dérivé de triazolo(4,5-d)pyrimidine de formule(I) selon la revendication 2.

14. Méthode d'élimination de bactéries dans la formation de biofilm sur une surface selon la revendication 13, où la formation de biofilm est dans une étape de maturation 3 comprenant plus de 90 x 10⁶ UFC/cm².

15. Méthode selon l'une quelconque des revendications 2 et 9 à 14, où la surface appartient à un biomatériau.

16. Méthode selon la revendication 15, où le biomatériau est un dispositif cardio-vasculaire.

17. Méthode selon la revendication 16, où le dispositif cardio-vasculaire est une valve cardiaque prothétique.

18. Méthode selon la revendication 16, où le dispositif est un stimulateur cardiaque.
